# FASCICULE DE BREVET EUROPEEN

(11) **EP 2 310 358 B1**
(45) Date de publication et mention de la délivrance du brevet: **26.09.2018**
(21) Numéro de dépôt: 09765751.4
(22) Date de dépôt: 05.06.2009
(51) Int. Cl.: C07C 253/10, B01J 31/24, C07C 255/04, B01J 31/18

(54) **PROCEDE DE FABRICATION DE COMPOSES NITRILES A PARTIR DE COMPOSES A INSATURATION ETHYLENIQUE**
VERFAHREN ZUR HERSTELLUNG VON NITRILVERBINDUNGEN AUS ETHYLENISCH UNGESÄTTIGTEN VERBINDUNGEN
PROCESS FOR PRODUCING NITRILE COMPOUNDS FROM ETHYLENICALLY UNSATURATED COMPOUNDS

(30) Priorité: 17.06.2008 FR 0803374
(43) Date de publication de la demande: 20.04.2011
(73) Titulaire: INVISTA Textiles (U.K.) Limited, Manchester M2 3DE (GB)
(72) Inventeur: MASTROIANNI, Sergio, 69006 Lyon (FR)
(74) Mandataire: Carpmaels & Ransford LLP
(86) Numéro de dépôt international: PCT/EP2009/056916
(87) Numéro de publication internationale: WO 2009/153171

(56) Documents cités:
- WO-A-02/053527
- FR-A- 2 830 530
- FR-A- 2 849 027
- US-A- 3 655 723
- US-A- 3 694 485
- US-A- 3 766 237
- US-A- 6 153 758

## Description

La présente invention concerne un procédé d'hydrocyanation en dinitriles de composés hydrocarboné comprenant au moins une insaturation éthylénique choisi parmi le pentène-3-nitrile, le pentène-4-nitrile et leurs mélanges.

Le brevet français n° 1 599 764 décrit un procédé de préparation de nitriles par addition d'acide cyanhydrique sur des composés organiques ayant au moins une double liaison éthylénique, en présence d'un catalyseur comprenant du nickel et un ligand organophosphorés, une triaryle phosphite. Cette réaction peut être conduite en présence ou non d'un solvant.

Quand un solvant est utilisé, il s'agit de préférence d'un hydrocarbure, tel que le benzène ou les xylènes ou d'un nitrile tel que l'acétonitrile.

Le catalyseur mis en oeuvre est un complexe organique de nickel, contenant des ligands tels que les phosphines, les arsines, les stibines, les phosphites, les arsénites ou les antimonites.

La présence d'un promoteur pour activer le catalyseur, tel qu'un composé du bore ou un sel métallique, généralement un acide de Lewis, est également préconisée dans ledit brevet.

De nombreux autres systèmes catalytiques ont été proposes comprenant généralement des composés organophosphorés appartenant à la famille des phosphites, phosphonites, phosphinites et phosphines. Ces composes organophosphorés peuvent comprendre un atome de phosphore par molécule et sont qualifiés de ligands monodentates. Ils peuvent comprendre plusieurs atomes de phosphore par molécule, ils sont alors appelés ligands pluridentates, plus particulièrement des ligands contenant deux atomes de phosphore par molécule (appelés ligand bidentate) ont été décrits dans de nombreux brevets. Le document US 3 766 237 décrit un procédé d'hydrocyanation utilisant un ligand triarylphosphite. Le document US 3 655 723 décrit un procédé d'hydrocyanation en présence d'un système catalytique comprenant du nickel et comme ligand une phosphine ou une phosphite. Le document US 3 694 485 décrit un procédé d'hydrocyanation en présence d'un système catalytique comprenant du fer ou du ruthénium, et comme ligand une phosphite ou une phosphine.

Toutefois, la recherche de nouveaux systèmes catalytiques plus performants tant en activité catalytique qu'en stabilité est toujours entreprise.

Un des buts de la présente invention est de proposer de nouveaux systèmes catalytiques qui présentent une bonne activité catalytique dans la réaction d'hydrocyanation.

A cet effet, la présente invention propose un procédé d'hydrocyanation d'un composé hydrocarboné comprenant au moins une insaturation éthylénique choisi parmi le pentène-3-nitrile, le pentène-4-nitrile et leurs mélanges par réaction en milieu liquide avec le cyanure d'hydrogène en présence d'un système catalytique comprenant le nickel, au moins un ligand organophosphoré formant un complexe avec le nickel, et un cocatalyseur consistant en au moins un acide de Lewis, caractérisé en ce que le système catalytique comprend au moins un composé organophosphite monodentate choisi dans le groupe comprenant les orthos, para, méta tritolylphosphites et leurs mélanges et d'au moins un composé organophosphine monodentate répondant à la formule générale (I). Le rapport molaire entre le composé organophosphite monodentate et le compose organophosphine monodentate est compris entre 0,01 et 100.

Le rapport molaire par rapport à un atome d'élément métallique pour le composé organophosphine est compris entre 0,1 et 10 et pour le composé organophosphite est compris entre 0,1 et 10.

Dans un premier mode de réalisation de l'invention, le rapport molaire entre le composé organophosphite et le composé organophosphine est avantageusement compris entre 1 et 100, préférentiellement entre 5 et 60.

Dans un second mode de réalisation de l'invention, le rapport molaire entre le composé organophosphite et le composé organophosphine est compris entre 0,01 et 0,3.

Selon une autre caractéristique applicable à tous les modes de réalisation de l'invention, le rapport molaire entre la somme des moles de composes organophosphine et organophoshite par rapport au nombre d'atome d'élément métallique est compris entre 4 et 10.

Les organophosphines convenables pour l'invention sont choisis dans le groupe comprenant les composes organophosphines répondant a la formule générale (I) : 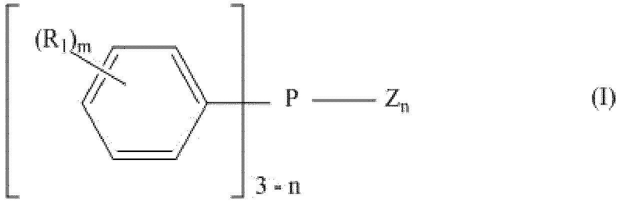 dans laquelle :
- Z représente un groupement thiényl tel que la liaison avec le phosphore est portée par le carbone en alpha de l'hétéroatome
- n représente un nombre entier de 1 à 3
- m représente un nombre entier de 0 à 5
- le radical R₁ représente un atome d'hydrogène, un radical alkyl linéaire ou ramifié pouvant contenir des hétéroatomes ayant de 1 à douze atomes de carbone, un radical aromatique ou cycloaliphatique substitué ou non pouvant comprendre des hétéroatomes, un radical carbonyle, alcoxycarbonyle ou alcoxy, un atome d'halogène, un groupement nitrile ou un groupement halogénoalkyle ayant de un à douze atomes de carbone.

Selon l'invention, la composition du système catalytique est illustrée par la formule générale (II) (Cette formule ne représente pas la structure des composes ou complexes présents dans le système catalytique) :

M[L₁]ₜ[L₂]ᵤ (II)

Dans laquelle:
M représente le nickel,
L₁ représente le ligand organophosphite,
L₂ représente le ligand organophosphine,
t et u différents ou identiques représentent un nombre compris entre 0, 1 et 9,9 bornes incluses,
la somme t+u étant comprise entre 4 et 10.

Le nickel est l'élément préfère pour l'hydrocyanation des oléfines et des nitriles insaturés.

Comme composes de formule générale (I) convenables, on peut citer à titre d'exemples non limitatifs, la (2-thiényl)diphénylphosphine, di(2-thiényl)phénylphosphine, la tri(2-thiényl)phosphine.

Pour la préparation des thienylphosphines selon la formule générale (I) on peut se référer par exemple a l'article de V.K. Issleib et A. Brack publié dans Zeitschrift für anorganische und allgemeine Chemie, 1957, 292, pages 245 à 253.

La préparation des complexes organométalliques comprenant des ligands organophosphorés convenables pour l'invention peut être effectuée en mettant en contact une
solution d'un composé du nickel, avec une solution du ou des composes organophosphorés de l'invention.

Le composé du nickel peut être dissous dans un solvant. Le nickel peut se trouver dans le compose mis en oeuvre, soit au degré d'oxydation qu'il aura dans le complexe organométallique, soit à un degré d'oxydation supérieur.

A titre d'exemple, on peut indiquer que dans les complexes organométalliques de l'invention, le nickel est au degré d'oxydation (0).

Si lors de la préparation du complexe organométallique, le nickel est mis en oeuvre à un degré d'oxydation plus élevé, ii pourra être réduit in situ.

Parmi les composés de nickel utilisables pour la préparation des complexés organométalliques on peut citer, à titre d'exemples non limitatifs, les composes suivants du nickel:
- les composés dans lesquels le nickel est au degré d'oxydation zéro comme le tetracyanonickelate de potassium K₄[Ni(CN)₄], le bis (acrylonitrile) nickel zéro, le bis (cyclooctadiène-1,5) nickel (appelé également Ni(cod)₂) et les dérives contenant des ligands comme le tétrakis (triphényl phosphine) nickel zéro.
- les composés du nickel comme les carboxylates (notamment l'acetate), carbonate, bicarbonate, borate, bromure, chlorure, citrate, thiocyanate, cyanure, formiate, hydroxyde, hydrophosphite, phosphite, phosphate et dérives, iodure, nitrate, sulfate, sulfite, aryl- et alkyl-sulfonates.

Quand le composé du nickel utilisé correspond à un état d'oxydation du nickel supérieur à 0, on ajoute au milieu réactionnel un réducteur du nickel réagissant préférentiellement avec celui-ci dans les conditions de la réaction. Ce réducteur peut être organique ou minéral. On peut citer comme exemples non limitatifs les borohydrures comme le BH₄Na, le BH₄K, la poudre de Zn, le magnésium ou l'hydrogène.

Quand le composé du nickel utilisé correspond à l'état d'oxydation 0 du nickel, on peut également ajouter un réducteur du type de ceux précités, mais cet ajout n'est pas impératif.

Les pentène-nitriles peuvent contenir, en plus du pentène-3-nitrile et du pentène-4-nitrile, des quantités, généralement minoritaires, d'autres composés, comme le méthyl-2-butène-3-nitrile, le méthyl-2-butène-2-nitrile, le pentène-2-nitrile, le valéronitrile, l'adiponitrile, le méthyl-2-glutaronitrile, l'éthyl-2-succinonitrile ou le butadiène, provenant par exemple de la réaction antérieure d'hydrocyanation du butadiène en nitriles insaturés.

En effet, lors de l'hydrocyanation du butadiène, il se forme avec les pentène-nitriles linéaires des quantités non négligeables de méthyl-2-butène-3-nitrile et de méthyl-2-butène 2-nitrile.

Le système catalytique utilisé pour l'hydrocyanation selon le procédé de l'invention peut être prépare avant son introduction dans la zone de réaction, par exemple par addition a la phosphine de formule (I) seule ou dissoute dans un solvant, la quantité appropriée de composé du métal de transition choisi et éventuellement du réducteur. Il est également possible de préparer le système catalytique "in situ" par simple addition de la phosphine et du compose du métal de transition dans le milieu réactionnel d'hydrocyanation avant ou après l'addition du composé à hydrocyaner.

La quantité de compose du nickel est choisie pour obtenir une concentration en mole de métal de transition par mole de composés organiques à hydrocyaner ou isomériser comprise entre 10⁻⁴ et 1, et de préférence entre 0,005 et 0,5 mole de nickel.

La quantité de composés organophosphorés utilisée pour former le système catalytique est choisie de telle sorte que le nombre de moles de ces composés rapporté à 1 mole de métal de transition soit de 0,5 à 100 et de préférence de 2 à 50.

Bien que la réaction soit conduite généralement sans solvant, il peut être avantageux de rajouter un solvant organique inerte. Le solvant peut être un solvant du catalyseur qui est miscible à la phase comprenant le composé à hydrocyaner à la température d'hydrocyanation. A titre d'exemples de tels solvants, on peut citer les hydrocarbures aromatiques, aliphatiques ou cycloaliphatiques.

La réaction d'hydrocyanation est généralement réalisée à une température de 10°C à 200°C et de préférence de 30°C à 120°C. Elle peut être réalisée en milieu monophasique.

Le procédé de l'invention peut être mis en oeuvre de manière continue ou discontinue.

Le cyanure d'hydrogène mis en oeuvre peut être préparé à partir des cyanures métalliques, notamment le cyanure de sodium, ou des cyanhydrines, comme la cyanhydrine de l'acétone ou par tout autre procédé de synthèse connu tel que le procédé Andrussov consistant à faire réagir le méthane avec de l'ammoniac et de l'air.

Le cyanure d'hydrogène exempt d'eau est introduit dans le réacteur sous forme gazeuse ou sous forme liquide. Il peut également être préalablement dissous dans un solvant organique.

Dans le cadre d'une mise en oeuvre discontinue, on peut en pratique charger dans un réacteur, préalablement purgé à l'aide d'un gaz inerte (tel qu'azote, argon), soit une solution contenant la totalité ou une partie des divers constituants tels que les composés oraganophosphorés convenables pour l'invention, le composé de métal de transition (le nickel), les éventuels réducteur et solvant, soit séparément lesdits constituants. Généralement le réacteur est alors porté à la température choisie, puis le composé à hydrocyaner est introduit. Le cyanure d'hydrogène est alors lui-même introduit, de préférence de manière continue et régulière.

Quand la réaction (dont on peut suivre l'évolution par dosage de prélèvements) est terminée, le mélange réactionnel est soutiré après refroidissement et les produits de la réaction sont isolés et séparés, par exemple, par distillation.

Avantageusement, la synthèse de dinitriles tels que l'adiponitrile à partir de dioléfines (butadiène) est obtenue en deux étapes successives. La première étape consiste à hydrocyaner une double liaison de la dioléfine pour obtenir un mononitrile insaturé. La seconde étape consiste à hydrocyaner l'insaturation du mononitrile pour obtenir le ou les dinitriles correspondants et peut répondre au procédé de l'invention. Ces deux étapes sont généralement mises en oeuvre avec un système catalytique comprenant un complexe organométallique de même nature. Toutefois, les rapports composes organophosphorés/ élément métallique et concentration du catalyseur peuvent être différents. En outre, il est associé au système catalytique un cocatalyseur dans la seconde étape. Ce cocatalyseur est un acide de Lewis.

L'acide de Lewis utilisé comme cocatalyseur permet notamment, dans le cas de l'hydrocyanation des nitriles aliphatiques à insaturation éthylénique, d'améliorer la linéarité des dinitriles obtenus, c'est-à-dire le pourcentage de dinitrile linéaire par rapport à la totalité des dinitriles formes, et/ou d'augmenter l'activité et la durée de vie du catalyseur.

Par acide de Lewis, on entend dans le présent texte, selon la définition usuelle, des composés accepteurs de doublets électroniques.

On peut mettre en oeuvre notamment les acides de Lewis cités dans l'ouvrage édité par G.A. OLAH "Friedel-Crafts and related Reactions", tome I, pages 191 à 197 (1963).

Les acides de Lewis qui peuvent être mis en oeuvre comme cocatalyseurs dans le présent procédé sont choisis parmi les composés des éléments des groupes Ib, IIb, IIIa, IIIb, IVa, IVb, Va, Vb, VIb, VIIb et VIII de la Classification périodique des éléments. Ces composes sont le plus souvent des sels, notamment des halogénures, comme chlorures ou bromures, sulfates, sulfonates, halogenosulfonates, perhalogénoalkyl sulfonates, notamment fluoroalkylsulfonates ou perfluoroalkylsulfonates, carboxylates et phosphates.

A titre d'exemples non limitatifs de tels acides de Lewis, on peut citer le chlorure de zinc, le bromure de zinc, l'iodure de zinc, le chlorure de manganèse, le bromure de manganèse, le chlorure de cadmium, le bromure de cadmium, le chlorure stanneux, le bromure stanneux, le sulfate stanneux, le tartrate stanneux, le trifluorométhylsulfonate d'indium, les chlorures ou bromures des éléments des terres rares comme le lanthane, le cérium, le praséodyme, le néodyme, le samarium, l'europium, le gadolinium, le terbium, le dysprosium, l'hafnium, l'erbium, le thallium, l'ytterbium et le lutétium, le chlorure de cobalt, le chlorure ferreux, le chlorure d'yttrium, les composés décrits dans le brevet français n°2926816.

On peut également utiliser comme acide de Lewis des composés organométalliques comme le triphénylborane, l'isopropylate de titane.

On peut bien entendu mettre en oeuvre des mélanges de plusieurs acides de Lewis.

Parmi les acides de Lewis, on préfère tout particulièrement le chlorure de zinc, le bromure de zinc, le chlorure stanneux, le bromure stanneux le triphénylborane et les mélanges chlorure de zinc/chlorure stanneux.

Le cocatalyseur acide de Lewis mis en oeuvre représente généralement de 0,01 à 50 moles par mole de composé du nickel, et de préférence de 1 à 10 mole par mole.

Les mononitriles insaturés mis en oeuvre dans cette deuxième étape sont des pentène-nitriles linéaires choisi parmi le pentène-3-nitrile, le pentène-4-nitrile et leurs mélanges.

Ces pentène-nitriles peuvent contenir des quantités, généralement minoritaires, d'autres composes, comme le méthyl-2-butène-3-nitrile, le méthyl-2-butène-2-nitrile, le pentène-2-nitrile.

La solution catalytique utilisée pour l'hydrocyanation en présence d'acide de Lewis peut être préparée avant son introduction dans la zone de réaction, par exemple par addition a la phosphine de formule (I), de la quantités appropriée de composé du nickel, de l'acide de Lewis et éventuellement du réducteur. Il est également possible de préparer la solution catalytique "in situ" par simple addition de ces divers constituants dans le milieu réactionnel.

Il est également possible dans les conditions du procédé d'hydrocyanation, et notamment en opérant en présence du catalyseur décrit précédemment comportant au moins un composé de formule (I) et au moins un composé d'un métal de transition, de réaliser, en absence de cyanure d'hydrogène, l'isomérisation du méthyl-2-butène-3-nitril en pentènenitriles, et plus généralement des nitriles insaturés ramifies en nitriles insaturés linéaires.

Le méthyl-2-butène-3-nitril soumis à l'isomérisation peut être mis en oeuvre seul ou en mélange avec d'autres composés. Ainsi on peut engager du méthyl-2-butène-3-nitril en mélange avec du méthyl-2-butène-2-nitril, du pentène-4-nitrile, du pentène-3-nitrile, du pentène-2-nitrile, du butadiène

Il est particulièrement intéressant de traiter le mélange réactionnel provenant de l'hydrocyanation du butadiène par HCN en présence d'au moins un composé de formule (I) et d'au moins un composé d'un métal de transition, plus préférentiellement d'un composé du nickel au degré d'oxydation 0, tel que défini précédemment. Dans le cadre de cette variante, le system catalytique étant déjà présent pour la réaction d'hydrocyanation du butadiène, il suffit d'arrêter toute introduction de cyanure d'hydrogène, pour laisser se produire la réaction d'isomérisation. On peut, le cas échéant, dans cette variante faire un léger balayage du réacteur a l'aide d'un gaz inerte comme l'azote ou l'argon par exemple, afin de chasser l'acide cyanhydrique qui pourrait être encore présent.

La réaction d'isomérisation est généralement réalisée à une température comprise entre 10°C et 200° C et de préférence entre 60° C et 140° C.

Dans le cas d'une isomérisation suivant immédiatement la réaction d'hydrocyanation du butadiène, ii sera avantageux d'opérer à la température à laquelle l'hydrocyanation a été conduite ou légèrement supérieure.

Comme pour le procédé d'hydrocyanation de composés a insaturation éthylénique, le system catalytique utilisé pour l'isomérisation peut être prépare avant son introduction dans la zone de réaction, par exemple par mélange du composé de formule (I), de la quantité appropriée de composé du métal de transition choisi et éventuellement du réducteur. Il est également possible de préparer le system catalytique "in situ" par simple addition de ces divers constituants dans le milieu réactionnel. La quantité de composé du métal de transition et plus particulièrement du nickel utilisée, ainsi que la quantité de composé de formule (I) sont les mêmes que pour la réaction d'hydrocyanation.

Bien que la réaction d'isomérisation soit conduite généralement sans solvant, il peut être avantageux de rajouter un solvant organique inerte qui pourra être utilisé comme solvant d'extraction, ultérieurement. C'est notamment le cas lorsqu'un tel solvant a été mis en oeuvre dans la réaction d'hydrocyanation du butadiène ayant servi à préparer le milieu soumis à la réaction d'isomérisation. De tels solvants peuvent être choisis parmi ceux qui ont été cités précédemment pour l'hydrocyanation.

Toutefois, la préparation de composés dinitriles par hydrocyanation d'une oléfine comme le butadiène peut être réalisée en utilisant un système catalytique conforme a l'invention pour les étapes de formation des nitriles insaturés et l'étape d'isomérisation ci-dessus.

De même, la réaction d'hydrocyanation de l'oléfine en nitriles insaturés et l'isomérisation de ceux-ci peuvent être réalisées avec un système catalytique différent de celui de l'invention, l'étape d'hydrocyanation des nitriles insaturés en dinitriles étant mis en oeuvre avec un système catalytique conforme à l'invention.

D'autres détails, avantages de l'invention seront illustrés par les exemples donnés ci-dessous uniquement à titre indicatif, sans caractère limitatif.

### Abréviations utilisées

- Cod: cyclooctadiène
- Ni(Cod)₂ : bis(1,5-cyclooctadiène)nickel
- 3PN: 3-penténenitrile
- AdN: Adiponitrile
- ESN: éthylsuccinonitrile
- MGN: méthylglutaronitrile
- ON : composés dinitriles (AdN, MGN OU ESN)
- TIBAO: tetraisobutyldialuminoxane
- Mes: groupement mésityl (2,4,6 triméthylphényl)
- Ph: groupement phényle
- TTP : tritolylphosphite
- TPP : triphénylphosphite
- RR(DN): rendement réel de dinitriles correspondant au rapport du nombre de moles formées de dinitriles sur le nombre de moles de 3PN engagé
- Linéarité (L): rapport du nombre de moles d'AdN formées sur le nombre de moles de dinitriles formées (somme des moles d'AdN, ESN et MGN)

Les composés suivants: 3PN, Ni(cod)₂, ZnC_{I2}, TTP, TPP, tris(2-thiényl)phosphine, tris(2-furyl)phosphine, tris(4-chlorophényl)phosphine sont des produits connus et disponibles

### Exemples 1 à 12 : Hydrocyanation du 3-PN en AdN

Les essais sont réalisés selon le mode opératoire suivant

Sous atmosphère d'argon, dans un tube de verre type Shott de 60 ml équipé d'un bouchon-septum, sont chargés successivement:
- ligand 1 (voir tableau 1 pour nature et quantité)
- ligand 2 (voir tableau 1 pour nature et quantité)
- 1,21 g (15 mmol, 30 équivalents) de 3PN
- 138 mg (0,5 mmol, 1 équivalent) de Ni(cod)₂
- 62 mg (0,5 mmol, 1 équivalent) de ZnCl₂

Le mélange est porté sous agitation à 70°C. La cyanhydrine de l'acétone est injectée dans le milieu réactionnel par un pousse-seringue à un débit de 0,45 ml par heure. Après 3 heures d'injection le poussé seringue est arrêté. Le mélange est refroidi à température ambiante, dilué à l'acétone et analysé par chromatographie en phase gazeuse.

Dans ces exemples, les ligands phosphines utilisées ont les formules III à V suivantes :

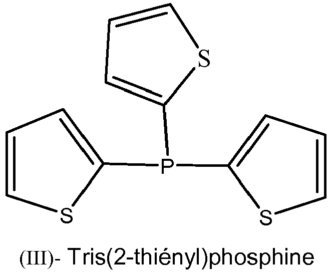

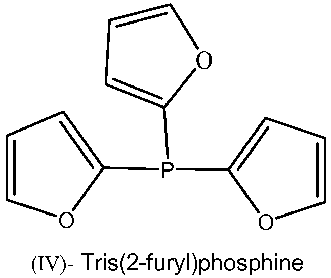

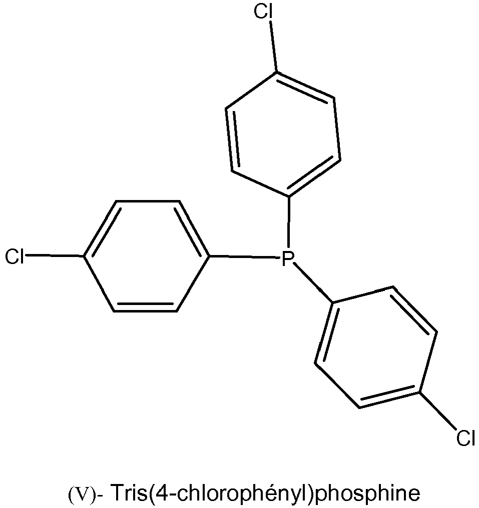

Les résultats sont regroupés dans le tableau 1 suivant

**Tableau 1 : exemples 1 à 14**

| Exemple | Ligand 1 | Ligand 2 | Ligand1/Ligand2/Ni (équivalents molaire) | Linéarité (L) | RR (DN) |
|---|---|---|---|---|---|
| 1 (comparatif) | TTP | - | 5/0/1 | 82.4 | 58,5 |
| 2 (comparatif) | Tris(2-thiényl)phosphine | - | 5/0/1 | 62,5 | 75,8 |
| 3 (comparatif) | Tris(4-chlorophényl)phosphine | - | 5/0/1 | 60 | 64,8 |
| 4 (comparatif) | Tris(2-furyl)phosphine | - | 5/0/1 | 64,6 | 78,4 |
| 5 | Tris(2-thiényl)phosphine | TTP | 1/4/1 | 77,3 | 71,5 |
| 6 | Tris(2-thiényl)phosphine | TTP | 2,5/2,5/1 | 72,8 | 75,9 |
| 7 | Tris(2-thiényl)phosphine | TTP | 4/1/1 | 67,8 | 79,3 |
| 8 (comparatif) | Tris(4-chlorophényl)phosphine | TTP | 2,5/2,5/1 | 75,9 | 67,2 |
| 9 (comparatif) | Tris(2-furyl)phosphine | TTP | 2,5/2,5/1 | 67,3 | 80,7 |
| 10 | Tris(2-thiényl)phosphine | TTP | 0,1/4,9/1 | 82,6 | 64,5 |
| 11 (comparatif) | Tris(4-chlorophényl)phosphine | TTP | 0,1/4,9/1 | 82,6 | 62,2 |
| 12 (comparatif) | Tris(2-furyl)phosphine | TTP | 0,1/4,9/1 | 81,6 | 62,8 |
| 13 (comparatif) | TTP | - | 5/0/1 | 81,9 | 33,8 |
| 14 | Tris(2-thiényl)phosphine | TTP | 0,1/4,9/1 | 80,7 | 38,5 |

## Revendications

1. Procédé d'hydrocyanation en dinitriles d'un composé hydrocarboné comprenant au moins une insaturation éthylénique choisi parmi le pentène-3-nitrile, le pentène-4-nitrile et leurs mélanges par réaction en milieu liquide avec le cyanure d'hydrogène en présence d'un système catalytique comprenant le nickel, au moins un ligand organophosphoré formant un complexe avec le nickel, et un cocatalyseur consistant en au moins un acide de Lewis, **caractérise en ce que** le système catalytique comprend au moins un composé organophosphite monodentate choisi dans le groupe comprenant les orthos, para, méta tritolylphosphites et leurs mélanges, au moins un composé organophosphine monodentate, le rapport molaire par rapport à un atome d'élément métallique pour le composé organophosphine est compris entre 0,1 et 10, et pour le composé organophosphite monodentate est compris entre 0,1 et 10, le rapport molaire entre le composé organophosphite et le composé organophosphine étant compris entre 0,01 et 100, le compose organophosphine étant un compose répondant à la formule générale (I): 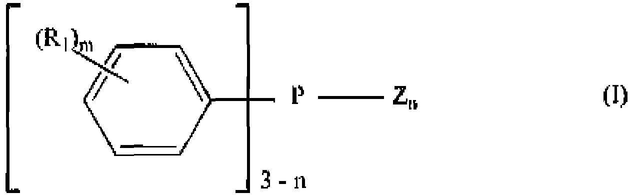 dans laquelle:
- Z représente un groupement thiényl tel que la liaison avec le phosphore est portée par le carbone en alpha de l'hétéroatome
- n représente un nombre entier de 1 à 3
- m représente un nombre entier de 0 à 5
- le radical R₁ représente un atome d'hydrogène, un radical alkyl linéaire ou ramifié pouvant contenir des hétéroatomes ayant de 1 à douze atomes de carbone, un radical aromatique ou cycloaliphatique substitute ou non pouvant comprendre des hétéroatomes, un radical carbonyle, alcoxycarbonyle ou alcoxy, un atome d'halogène, un groupement nitrile ou un groupement halogénoalkyle ayant de un à douze atomes de carbone.

2. Procédé selon la revendication 1, **caractérise en ce que** le rapport molaire entre le composé organophosphite et le composé organophosphine est compris entre 1 et 100.

3. Procédé selon la revendication 1, **caractérise en ce que** le rapport molaire entre le composé organophosphite et le composé organophosphine est compris entre 0,01 et 0,3.

4. Procédé selon l'une des revendications précédentes, **caractérisé en ce que** le rapport molaire entre la somme des moles de composés organophosphine et organophosphite par rapport au nombre d'atome d'élément métallique est compris entre 4 et 10.

5. Procédé selon l'une des revendications précédentes, **caractérisé en ce que** le composé de formule générale I répond à la formule III suivante:

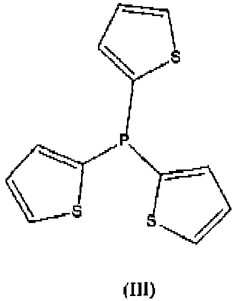

6. Procédé selon l'une des revendications précédentes, **caractérisé en ce que** la composition du système catalytique est illustrée par la formule générale (II):
M[L₁]ₜ[L₂]ᵤ (II)
dans laquelle:
M représente le nickel,
L₁ représente le ligand organophosphite,
L₂ représente le ligand organophosphine,
t et u différents ou Identiques représentent un nombre compris entre 0,1 et 9.9 bornes incluses,
la somme t+u étant comprise entre 4 et 10.

7. Procédé selon l'une des revendications précédentes, **caractérisé en ce que** la quantité de nickel utilisée est choisie de telle sorte qu'il y alt par mole de composé organique à hydrocyaner entre 10⁻⁴ et 1 mole de nickel mis en oeuvre.

8. Procédé selon l'une des revendications précédentes, **caractérisé en ce que** l'acide de Lewis mis en oeuvre comme cocatalyseur est choisi parmi les composés des éléments des groupes Ib, IIb, IIIa, IIIb, IVa, IVb, Va, Vb, VIb, VIIb et VIII de la Classification périodique des éléments.

9. Procédé selon la revendication 8, **caractérisé en ce que** l'acide de Lewis est choisi parmi le chlorure de zinc, le bromure de zinc, l'Iodure de zinc, le chlorure de manganèse, le bromure de manganèse, le chlorure de cadmium, le bromure de cadmium, le chlorure stanneux, le bromure stanneux, le sulfate stanneux, le tartrate stanneux, le trifluorométhylsulfonate d'indium, les chlorures ou bromures des éléments des terres rares comme le lanthane, le cérium, le praséodyme, le néodyme, le samarium, l'europium, le gadolinium, le terbium, le dysprosium, l'hafnium, l'erblum, le thallium, l'ytterbium et le lutetium, le chlorure de cobalt, le chlorure ferreux, le chlorure d'yttrlum et leurs melanges, les composés organométalliques.

## Patentansprüche

1. Verfahren zur Hydrocyanierung einer Kohlenwasserstoffverbindung mit mindestens einer ethylenischen Ungesättigtheiten, die aus 3-Pentennitril, 4-Pentennitril und Mischungen davon ausgewählt sind, zu Dinitrilverbindungen durch Umsetzung mit Cyanwasserstoff in flüssigem Medium in Gegenwart eines katalytischen Systems, das Nickel, mindestens einen Organophosphorliganden, der mit dem Nickel einen Komplex bildet, und einen Cokatalysator, der aus mindestens einer Lewis-Säure besteht, umfasst, **dadurch gekennzeichnet, dass** das katalytische System mindestens eine einzähnige Organophosphitverbindung, die aus der Gruppe umfassend ortho-, para- und meta-Tritolylphosphit und Mischungen davon ausgewählt ist, und mindestens eine einzähnige Organophosphinverbindung umfasst und das Molverhältnis in Bezug auf ein Metallelement-Atom für die Organophosphinverbindung zwischen 0,1 und 10 liegt und für die einzähnige Organophosphitverbindung zwischen 0,1 und 10 liegt, wobei das Molverhältnis zwischen der Organophosphitverbindung und der Organophosphinverbindung zwischen 0,01 und 100 liegt, wobei sich bei der Organophosphinverbindung um eine Verbindung handelt, die der allgemeinen Formel (I) entspricht: 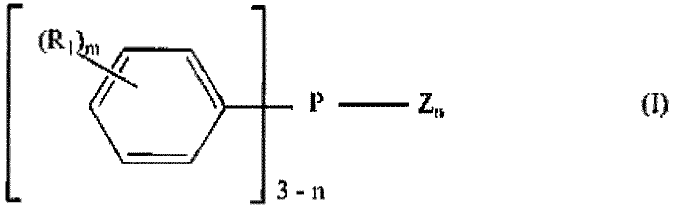 worin:
- Z für eine Thienylgruppe steht, so dass die Bindung mit dem Phosphor über den Kohlenstoff in der alpha-Position zum Heteroatom erfolgt,
- n für eine ganze Zahl von 1 bis 3 steht,
- m für eine ganze Zahl von 0 bis 5 steht,
- der Rest R₁ für ein Wasserstoffatom, einen linearen oder verzweigten Alkylrest, der Heteroatome enthalten kann und 1 bis zwölf Kohlenstoffatome aufweist, einen substituierten oder unsubstituierten aromatischen oder cycloaliphatischen Rest, der Heteroatome umfassen kann, einen Carbonyl-, Alkoxycarbonyl- oder Alkoxyrest, ein Halogenatom, eine Nitrilgruppe oder eine Halogenalkylgruppe mit eins bis zwölf Kohlenstoffatomen steht.

2. Verfahren nach Anspruch 1, **dadurch gekennzeichnet, dass** das Molverhältnis zwischen der Organophosphitverbindung und der Organophosphinverbindung zwischen 1 und 100 liegt.

3. Verfahren nach Anspruch 1, **dadurch gekennzeichnet, dass** das Molverhältnis zwischen der Organophosphitverbindung und der Organophosphinverbindung zwischen 0,01 und 0,3 liegt.

4. Verfahren nach einem der vorhergehenden Ansprüche, **dadurch gekennzeichnet, dass** das Molverhältnis zwischen der Summe der Mole von Organophosphin- und Organophosphitverbindung in Bezug auf die Zahl von Metallelement-Atomen zwischen 4 und 10 liegt.

5. Verfahren nach einem der vorhergehenden Ansprüche, **dadurch gekennzeichnet, dass** die Verbindung der allgemeinen Formel I der folgenden Formel III entspricht:

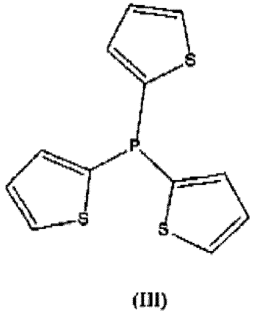

6. Verfahren nach einem der vorhergehenden Ansprüche, **dadurch gekennzeichnet, dass** die Zusammensetzung des Katalysators durch die allgemeine Formel (II) veranschaulicht wird:
M[L₁]ₜ[L₂]ᵤ (II)
worin:
M für Nickel steht,
L₁ für den Organophosphitliganden steht,
L₂ für den Organophosphinliganden steht,
t und u verschieden oder gleich sind und für eine Zahl zwischen 0,1 und 9,9 einschließlich der Grenzwerte stehen,
wobei die Summe t + u zwischen 4 und 10 liegt.

7. Verfahren nach einem der vorhergehenden Ansprüche, **dadurch gekennzeichnet, dass** man die verwendete Nickelmenge so gewählt, dass pro Mol zu hydrocyanierende organische Verbindung zwischen 10⁻⁴ und 1 mol Nickel verwendet werden.

8. Verfahren nach einem der vorhergehenden Ansprüche, **dadurch gekennzeichnet, dass** man die als Cokatalysator verwendete Lewis-Säure aus Verbindungen von Elementen der Gruppen Ib, IIb, IIIa, IIIb, IVa, IVb, Va, Vb, VIb, VIIb und VIII des Periodensystems der Elemente auswählt.

9. Verfahren nach Anspruch 8, **dadurch gekennzeichnet, dass** man die Lewis-Säure aus Zinkchlorid, Zinkbromid, Zinkiodid, Manganchlorid, Manganbromid, Cadmiumchlorid, Cadmiumbromid, Zinn(II)-chlorid, Zinn(II)-bromid, Zinn(II)-sulfat, Zinn(II)-tartrat, Indiumtrifluormethylsulfonat, den Chloriden oder Bromiden der Seltenerdmetalle wie Lanthan, Cer, Praseodym, Neodym, Samarium, Europium, Gadolinium, Terbium, Dysprosium, Hafnium, Erbium, Thallium, Ytterbium und Lutetium, Cobaltchlorid, Eisen(II)-chlorid, Yttriumchlorid und Mischungen davon und Organometallverbindungen auswählt.

## Claims

1. Process for the hydrocyanation, to give dinitriles, of a hydrocarbon-based compound comprising at least one ethylenic unsaturation chosen from 3-pentenenitrile, 4-pentenenitrile, and mixtures thereof, by reaction, in a liquid medium, with hydrogen cyanide in the presence of a catalytic system comprising nickel, at least one organophosphorus ligand which forms a complex with the nickel, and a cocatalyst consisting of at least one Lewis acid, **characterized in that** the catalytic system comprises at least one monodentate organophosphite compound chosen from the group comprising ortho-tritolyl phosphite, para-tritolyl phosphite and meta-tritolyl phosphite, and mixtures thereof, at least one monodentate organophosphine compound, the molar ratio relative to an atom of metal element, for the organophosphine compound, is between 0.1 and 10, and for the monodentate organophosphite compound, is between 0.1 and 10, the molar ratio of the organophosphite compound to the organophosphine compound being between 0.01 and 100, the organophosphine compound being a compound corresponding to the general formula (I): 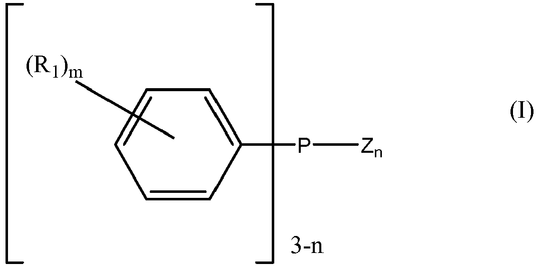 in which:
- Z represents a thienyl group such that the bond with the phosphorus is borne by the carbon in the alpha-position with respect to the heteroatom,
- n represents an integer from 1 to 3,
- m represents an integer from 0 to 5,
- the R₁ radical represents a hydrogen atom, a linear or branched alkyl radical, that may contain heteroatoms, having from 1 to twelve carbon atoms, a substituted or unsubstituted aromatic or cycloaliphatic radical that may comprise heteroatoms, a carbonyl, alkoxycarbonyl or alkoxy radical, a halogen atom, a nitrile group or a haloalkyl group having from one to twelve carbon atoms.

2. Process according to Claim 1, **characterized in that** the molar ratio of the organophosphite compound to the organophosphine compound is between 1 and 100.

3. Process according to Claim 1, **characterized in that** the molar ratio of the organophosphite compound to the organophosphine compound is between 0.01 and 0.3.

4. Process according to one of the preceding claims, **characterized in that** the molar ratio between the sum of the moles of organophosphine and organophosphite compounds and the number of atoms of metal element is between 4 and 10.

5. Process according to one of the preceding claims, **characterized in that** the compound of general formula I corresponds to the formula III below:

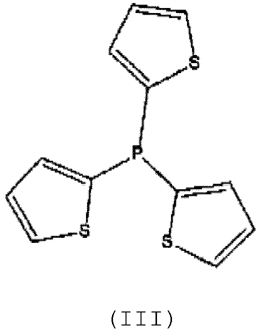

6. Process according to one of the preceding claims, **characterized in that** the composition of the catalytic system is illustrated by general formula (II):
M[L₁]ₜ[L₂]ᵤ (II)
in which:
M represents nickel,
L₁ represents the organophosphite ligand,
L₂ represents the organophosphine ligand,
t and u, which may be identical or different, represent a number between 0.1 and 9.9 (limits included),
the sum t+u being between 4 and 10.

7. Process according to one of the preceding claims, **characterized in that** the amount of nickel used is chosen such that between 10⁻⁴ and 1 mol of nickel are used per mole of organic compound to be hydrocyanated.

8. Process according to one of the preceding claims, **characterized in that** the Lewis acid used as cocatalyst is chosen from the compounds of elements from groups Ib, IIb, IIIa, IIIb, IVa, IVb, Va, Vb, VIb, VIIb and VIII of the Periodic Table of Elements.

9. Process according to Claim 8, **characterized in that** the Lewis acid is chosen from zinc chloride, zinc bromide, zinc iodide, manganese chloride, manganese bromide, cadmium chloride, cadmium bromide, stannous chloride, stannous bromide, stannous sulfate, stannous tartrate, indium trifluoromethylsulfonate, chlorides or bromides of rare earth elements such as lanthanum, cerium, praseodymium, neodymium, samarium, europium, gadolinium, terbium, dysprosium, hafnium, erbium, thallium, ytterbium and lutetium, cobalt chloride, ferrous chloride, yttrium chloride, and mixtures thereof, organometallic compounds.
